# EUROPEAN PATENT SPECIFICATION

(11) **EP 3 636 308 B1**
(45) Date of publication and mention of the grant of the patent: **25.08.2021**
(21) Application number: 17912646.1
(22) Date of filing: 13.06.2017
(51) Int. Cl.: A61M 16/04, A61M 16/08

(54) **EFFICIENT CARE-TYPE VARIABLE ENDOTRACHEAL TUBE VENTILATION DEVICE**
EFFIZIENTER VARIABLER ENDOTRACHEALTUBUSBELÜFTER FÜR MEDIZINISCHE VERSORGUNG
DISPOSITIF DE VENTILATION DE TUBE ENDOTRACHÉAL VARIABLE DE TYPE SOIN EFFICACE

(30) Priority: 06.06.2017 CN 201710420898
(43) Date of publication of application: 15.04.2020
(73) Proprietor: Xiao, Jinfang, Guangzhou, Guangdong 510000 (CN)
(72) Inventor: XIAO, Ming, Guangzhou Guangdong 510000 (CN); XIAO, Jinfang, Guangzhou Guangdong 510000 (CN)
(74) Representative: Cabinet Nuss
(86) International application number: PCT/CN2017/088123
(87) International publication number: WO 2018/223409

(56) References cited:
- WO-A1-2016/124879
- WO-A2-2015/013377
- CN-A- 102 824 676
- CN-A- 105 477 758
- CN-A- 105 579 090
- CN-A- 106 512 170
- CN-U- 202 078 625
- CN-U- 205 698 802
- US-A1- 2009 165 794
- US-A1- 2010 313 896
- US-A1- 2014 018 615
- US-A1- 2015 265 790
- US-A1- 2016 101 253

## Description

### Cross-reference to Related Application

The present disclosure claims the priority to the Chinese patent application with the filing number 201710420898.1 entitled "Efficient Care-type Variable Endotracheal Tube Ventilation Device" filed on June 6, 2017 with the Chinese Patent Office.

### Technical Field

The present disclosure relates to the technical field of medical instruments, particularly to an efficient care-type variable endotracheal tube ventilation device.

### Background Art

Endotracheal intubation refers to placing a special endotracheal tube into trachea through glottis, and this technology can provide optimal conditions for smooth airway, ventilation and oxygen supply, respiratory tract suction, aspiration prevention and the like.

In the above, the endotracheal tube in the prior art mainly includes: an endotracheal tube main body, and the endotracheal tube main body has one end as an air outlet, and the other end as an air inlet which can be connected with an oxygen tube. During use, the above endotracheal tube main body is inserted through the glottis into the upper respiratory track of the human body and fixed, meanwhile, the air inlet is connected to a breathing machine, so as to assist the patient in breathing, thereby meeting the requirement of the human body on oxygen exchange.

However, the inventor of the present disclosure has found that the endotracheal tubes in the prior art have relatively great stimulation to the airway of a patient, and easily cause suffering to the patient, and especially, the endotracheal tubes induce choking cough reflex when the patient is waking from general anesthesia.

Specifically, connection between the ventilation device and an anesthesia machine during the operation of the patient under the perioperative general anesthesia is mainly completed by a conventional endotracheal tube or a laryngeal mask. The application of the laryngeal mask is an important means for solving the hypersensitive reaction of airway of the patient under general anesthesia, but use of the laryngeal mask has its limitations, for example, about 4 hours of operation time, air leakage, inapplicability to patients with special body positions, inapplicability to patients with brain operations and the like, and the patients may be complicated by oropharyngeal cavity pains, hoarseness and wheezing after prolonged use. The conventional endotracheal tubes are still commonly used, but the stimulation of the endotracheal tube during the analepsia period of general anesthesia can cause severe choking cough reflex of the patient, and particularly some methods for avoiding postoperative choking cough reaction due to the endotracheal tube but causing brain injury after brain operation attract attention in recent years. Propofol can relieve choking cough, but as all drugs, drug metabolism increases liver load; the use of opioid analgesics can effectively relieve the occurrence of pains, and reduce the choking cough reflex, but can possibly increase the incidence rate of postoperative nausea, vomiting and respiratory depression; dexmedetomidine can improve the patient's tolerance to the endotracheal tube during the analepsia period of general anesthesia, but the drug metabolism increases the body's load, and meanwhile influences circulation function; the above anesthetics are not beneficial to recovery of consciousness of the patient after the operation, and delay the analepsia of the patient. In the perioperative analepsia period of general anesthesia of a surgical patient, after the recovery of respiration and swallowing reflex, when sputum suction and extubation operation affect, tracheal mucosa is directly stimulated by the catheter, which may cause severe choking cough reaction, and the choking cough reaction causes dramatic hemodynamic fluctuation, increases concentrations of plasma epinephrine and noradrenaline, then the peripheral vascular resistance and pulmonary vascular resistance are increased, with clinical manifestations of tachypnea, increased heart rate, and sudden rise of blood pressure; the imbalance of the proportion of myocardial oxygen supply and oxygen consumption resulted therefrom further may cause complications such as myocardial ischemia, heart failure and arrhythmia; especially cardiovascular accidents may be induced for patients with a history of hypertension, diabetes; at the same time, the choking cough may cause bleeding at vulnus and wound, wound dehiscence and so on. Therefore, a series of complications caused by the choking cough reflex induced by the endotracheal tube during the analepsia period of general anesthesia are the key and difficult points of management of perioperative airway and are increasingly taken into consideration. The choking cough is characterized by generation of a huge pressure in the thoracic cavity and strong airflow, and the pressure in the thoracic cavity can be conducted to the wound surface through blood vessels, causing wound bleeding. Clinically, a plurality of medicines for preventing choking cough or relieving stress reaction of the endotracheal tube are researched, such as opioid analgesics, propofol, dexmedetomidine and so on; the anesthetics can relieve the patient from choking cough, but delay the waking of the patient and affect the waking quality, and increase the load of liver and kidney on drug metabolism.

Taking intracranial tumor operation as an example, patients have the choking cough reaction in the analepsia process or the sputum suction process, and the occurrence rate of the choking cough reaction is high mostly during the period of removing the endotracheal tube. The choking cough may cause bleeding of cerebral wound, brain swelling, cerebral hematoma and intracranial pressure rise, and may cause secondary brain injury (SBI). In clinical anesthesia, the response of airway of the body excited by the endotracheal tube is obvious during the analepsia period of general anesthesia, and severe choking cough response is caused, so that the concentrations of plasma epinephrine and noradrenaline are increased, then the peripheral vascular resistance and pulmonary vascular resistance are increased, and the unstable breathing and circulating systems are manifested by tachypnea, increased heart rate and sudden rise of blood pressure; the imbalance of the proportion of myocardial oxygen supply and oxygen consumption caused thereby further may cause myocardial ischemia, affect organ perfusion and tissue oxygenation, and may seriously harm patients complicated with hypertension, diabetes or suffered from heart diseases due to serious myocardial ischemia, heart failure, arrhythmia and other complications. Therefore, more and more attention is paid to research of airway management during craniocerebral operation and a series of pathophysiological responses of the patients due to the choking cough induced by the endotracheal tube after analepsia.

In clinical anesthesia of a patient with liver operation, the response of airway of the body excited by the endotracheal tube is obvious during the analepsia period of general anesthesia, severe choking cough response is caused, and wound is also easy to bleed. Other patients with hypertension, diabetes, obesity and apnea are also susceptible to associated complications.

Therefore, how to provide a safer efficient care-type variable endotracheal tube ventilation device, which can eliminate the stimulation of the endotracheal tube to the airway of the patient under the perioperative general anesthesia to the greatest extent, and effectively improve the tolerance of the patient, has become a technical problem which urgently needs to be solved by those skilled in the art.

US 2014/018615 A1 discloses a telescopic intubation tube with distal camera.

### Summary

An object of the present disclosure is to provide an efficient care-type variable endotracheal tube ventilation device, so as to solve the technical problems that the existing endotracheal tube has relatively great stimulation to the airway of the patient, and easily causes suffering to the patient.

An efficient care-type variable endotracheal tube ventilation device provided in the present invention is defined in the appended claims.

Specifically, when the variable endotracheal tube head part is in a cylindrical-shape ventilation state, the diameter of the variable endotracheal tube head part is the same as the diameter of the main tube of the telescopic stacked endotracheal tube.

Further, the variable endotracheal tube head part is provided with a side opening.

Furthermore, at least two side openings are included.

Optionally, the plurality of side openings are distributed in a triangle shape in a three-dimensional space.

Optionally, the plurality of side openings are distributed in a cross shape in a three-dimensional space.

Optionally, the plurality of side openings are distributed in a quadrilateral shape in a three-dimensional space.

In practical application, the telescopic stacked endotracheal tube has a main tube which is of an elastic film of PVC material or an elastic film of paper material with antibacterial property; and the PVC material or the paper material has a thickness of 0.07 mm-0.17 mm.

In the above, an extended length of the main tube of the telescopic stacked endotracheal tube is at least two times the length when the main tube of the telescopic stacked endotracheal tube is completely stacked; the endotracheal tube main tube is configured in such a manner that the stacks at a distal end of the endotracheal tube are preferentially released when the endotracheal tube is moved in a direction from oral cavity towards the interior of the trachea, and a proximal end of the endotracheal tube is preferentially stacked when the endotracheal tube is retracted in a direction from the interior of the trachea towards the oral cavity, with a stacking thickness of less than 1.3 mm.

Specifically, the main tube of the telescopic stacked endotracheal tube is provided therein with a guide tube, and the guide tube is provided with rail holes along an axial direction; the main tube of the telescopic stacked endotracheal tube is provided therein with a plurality of elastic supports spaced apart in sequence along an axial direction, and each of the elastic supports supports the main tube of the telescopic stacked endotracheal tube in a circumferential direction, and each elastic support has a diameter of 0.1 mm-0.2 mm; the guide tube is provided in a manner of passing through the plurality of elastic supports, and the endotracheal tube main body can slide on the guide tube in a telescopic stacking manner.

Further, the guide tube participates in ventilation, and a plurality of holes on a tube wall of the guide tube are all formed in a tear shape, participating in ventilation

Furthermore, each of the holes of the guide tube formed in a tear shape has a diameter of 1 mm in a head portion, and a diameter of 0.1-0.2 mm in a tail portion.

Preferably, the elastic support is a spring.

In practical application, the endotracheal tube plugging cuff is communicated with an inflation valve.

Compared with the prior art, the efficient care-type variable endotracheal tube ventilation device described in the present disclosure has the following advantages:
The efficient care-type variable endotracheal tube ventilation device provided in the present disclosure includes a telescopic endotracheal tube main body with a variable length, and the endotracheal tube main body includes: a main tube of the telescopic stacked endotracheal tube, and a variable endotracheal tube head part connected to one end of the main tube of the telescopic stacked endotracheal tube; the variable endotracheal tube head part is formed of a temperature control material, and the variable endotracheal tube head part is developed to a cylindrical shape when the temperature reaches more than 30 degrees centigrade or 54.6 degrees Fahrenheit (12.6 °C), and the variable endotracheal tube head part is retracted back to a conical shape when the temperature reaches lower than 10 degrees centigrade or 18.6 degrees Fahrenheit (-7.4 °C), or the variable endotracheal tube head part is in a stacked conical shape, and the stacked conical shape of the variable endotracheal tube head part can be developed to the cylindrical shape by an air flow aeration method; an endotracheal tube plugging cuff is sheathed outside the endotracheal tube main body, and the endotracheal tube plugging cuff is provided near the variable endotracheal tube head part; and the other end of the main tube of the telescopic stacked endotracheal tube is connected to an endotracheal tube connector It can be seen from the analysis that for the efficient care-type variable endotracheal tube ventilation device provided in the present disclosure, as a ventilation shape of the variable endotracheal tube head part can be changed, and the main tube of the telescopic stacked endotracheal tube can change the length of the endotracheal tube main body in a telescoping manner, the efficient care-type variable endotracheal tube ventilation device provided in the present disclosure can eliminate, to the greatest extent, the stimulation of the endotracheal tube to airway of a patient under the perioperative general anesthesia, effectively improve the tolerance of the patient, and is safer; besides, the patient with the tube can have a conversation while being awake, and in emergency circumstances, the endotracheal tube state can be restored or the endotracheal tube can be inserted again at any time, which is particularly suitable for patients who need to reserve the endotracheal tube when returning to the ward or ICU.

### Brief Description of Drawings

In order to more clearly illustrate specific embodiments of the efficient care-type variable endotracheal tube ventilation device of the present disclosure or technical solutions in the prior art, accompanying drawings which need to be used for description of the specific embodiments or the prior art will be introduced briefly below. Apparently, the accompanying drawings in the following description merely show some particular embodiments of the present disclosure. All of other accompanying drawings obtained by a person ordinarily skilled in the art in light of these accompanying drawings, without using inventive efforts, fall within the scope of protection of the present disclosure.
FIG. 1 is a structural schematic diagram of an efficient care-type variable endotracheal tube ventilation device, in a first working state, provided in an embodiment of the present disclosure;
FIG. 2 is a structural schematic diagram of the efficient care-type variable endotracheal tube ventilation device, in a second working state, provided in an embodiment of the present disclosure;
FIG. 3 is a structural schematic diagram of another efficient care-type variable endotracheal tube ventilation device provided in an embodiment of the present disclosure;
FIG. 4 is a structural schematic diagram of another efficient care-type variable endotracheal tube ventilation device, with a first telescopic length, provided in an embodiment of the present disclosure;
FIG. 5 is a structural schematic diagram of another efficient care-type variable endotracheal tube ventilation device, with a second telescopic length, provided in an embodiment of the present disclosure;
FIG. 6 is a structural schematic diagram of the efficient care-type variable endotracheal tube ventilation device, in an intubation state during anesthesia, provided in an embodiment of the present disclosure;
FIG. 7 is a structural schematic diagram of the efficient care-type variable endotracheal tube ventilation device, in a state that the catheter is retracted back to the oral cavity during partial recovery of consciousness, provided in an embodiment of the present disclosure; and
FIG. 8 is a structural schematic diagram of the efficient care-type variable endotracheal tube ventilation device, in a state that the catheter is removed during complete recovery of consciousness, provided in an embodiment of the present disclosure.

In the drawings: 1-endotracheal tube main body; 11-main tube of the telescopic stacked endotracheal tube; 12-variable endotracheal tube head part; 2-endotracheal tube plugging cuff; 3-endotracheal tube connector; 121-side opening; 4-guide tube; 41-rail hole; 5-elastic support; 6-inflation valve.

### Detailed Description of Embodiments

Technical solutions of an efficient care-type variable endotracheal tube ventilation device of the present disclosure will be described below clearly and completely in combination with accompanying drawings. Apparently, the embodiments described are only a part of embodiments of the present disclosure, rather than all embodiments. All other embodiments, obtained by those ordinarily skilled in the art based on the embodiments in the present disclosure without using inventive efforts, shall fall within the scope of protection of the present disclosure.

In the description of the present disclosure, it should be noted that orientation or positional relationships indicated by terms such as "center", "upper", "lower", "left", "right", "vertical", "horizontal", "inner", "outer" and so on are based on orientation or positional relationships as shown in the accompanying drawings, merely for facilitating describing the present disclosure and simplifying the description, rather than indicating or suggesting that related devices or elements have to be in the specific orientation or configured and operated in a specific orientation, therefore, they should not be construed as limiting the present disclosure. Besides, terms "first", "second", and "third" are merely for descriptive purpose, but should not be construed as indicating or implying relative importance.

In the description of the efficient care-type variable endotracheal tube ventilation device of the present disclosure, it should be noted that unless otherwise specified and defined explicitly, terms "mount", "link", and "connect" should be construed in a broad sense. For example, it may be fixed connection, detachable connection, or integral connection; it may be mechanical connection, and also may be electrical connection; it may be direct connection, indirect connection through an intermediate medium, or inner communication between two elements. For a person ordinarily skilled in the art, specific meanings of the above-mentioned terms in the present disclosure can be understood according to specific circumstances.

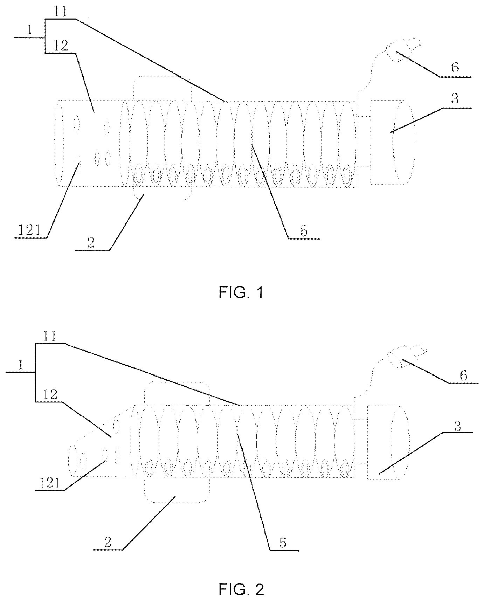

FIG. 1 is a structural schematic diagram of an efficient care-type variable endotracheal tube ventilation device, in a first working state, provided in an embodiment of the present disclosure; FIG. 2 is a structural schematic diagram of the efficient care-type variable endotracheal tube ventilation device, in a second working state, provided in an embodiment of the present disclosure

As shown in FIG. 1 and FIG. 2, an embodiment of the present disclosure provides an efficient care-type variable endotracheal tube ventilation device, including: a telescopic endotracheal tube main body 1 with a variable length, wherein the endotracheal tube main body 1 includes: a main tube of the telescopic stacked endotracheal tube 11, and a variable endotracheal tube head part 12 connected to one end of the main tube of the telescopic stacked endotracheal tube 11; the variable endotracheal tube head part 12 is formed of a temperature control material, and when the temperature reaches more than 30 degrees centigrade or 54.6 degrees Fahrenheit (12.6 °C), the variable endotracheal tube head part 12 is developed to a cylindrical shape, and when the temperature reaches lower than 10 degrees centigrade or 18.6 degrees Fahrenheit (-7.4 °C), the variable endotracheal tube head part 12 is retracted back to a conical shape; or the variable endotracheal tube head part 12 is in a stacked conical shape, and the stacked conical shape of the variable endotracheal tube head part 12 can be developed to the cylindrical shape by an air flow aeration method; an endotracheal tube plugging cuff 2 is sheathed outside the endotracheal tube main body 1, and the endotracheal tube plugging cuff 2 is provided near the variable endotracheal tube head part 12; and the other end of the main tube of the telescopic stacked endotracheal tube 11 is connected to an endotracheal tube connector 3.

Compared with the prior art, the efficient care-type variable endotracheal tube ventilation device described in the embodiment of the present disclosure has following advantages:
The efficient care-type variable endotracheal tube ventilation device provided in the embodiment of the present disclosure, as shown in FIG. 1 and FIG. 2, includes: the telescopic endotracheal tube main body 1 with a variable length, wherein the endotracheal tube main body 1 includes: the main tube of the telescopic stacked endotracheal tube 11, and the variable endotracheal tube head part 12 connected to one end of the main tube of the telescopic stacked endotracheal tube 11; the variable endotracheal tube head part 12 is formed of a temperature control material, and when the temperature reaches more than 30 degrees centigrade or 54.6 degrees Fahrenheit (12.6°C), the variable endotracheal tube head part 12 is developed to a cylindrical shape, and when the temperature reaches lower than 10 degrees centigrade or 18.6 degrees Fahrenheit (-7.4 °C), the variable endotracheal tube head part 12 is retracted back to a conical shape; or the variable endotracheal tube head part 12 is in a stacked conical shape, and the stacked conical shape of the variable endotracheal tube head part 12 can be developed to the cylindrical shape by an air flow aeration method; the endotracheal tube plugging cuff 2 is sheathed outside the endotracheal tube main body 1, and the endotracheal tube plugging cuff 2 is provided near the variable endotracheal tube head part 12; and the other end of the main tube of the telescopic stacked endotracheal tube 11 is connected to the endotracheal tube connector 3. It can be seen from the analysis that for the efficient care-type variable endotracheal tube ventilation device provided in the embodiment of the present disclosure, as a ventilation shape of the variable endotracheal tube head part 12 can be changed, and the main tube of the telescopic stacked endotracheal tube 11 can change the length of the endotracheal tube main body 1 in a telescoping manner, the efficient care-type variable endotracheal tube ventilation device provided in the embodiment of the present disclosure can eliminate, to the greatest extent, the stimulation of the endotracheal tube to airway of a patient under the perioperative general anesthesia, effectively improve the tolerance of the patient, and is safer; besides, the patient with the tube can have a conversation while being awake, and in emergency circumstances, the endotracheal tube state can be restored or the endotracheal tube can be inserted again at any time, which is particularly suitable for patients who need to reserve the endotracheal tube when returning to the ward or ICU.

In the above, in the efficient care-type variable endotracheal tube ventilation device provided in the embodiment of the present disclosure, the above variable endotracheal tube head part 12 can switch between a conical-shape ventilation state (as shown in FIG. 2) and a cylindrical-shape ventilation state (as shown in FIG. 1).

Specifically, as shown in FIG. 1, when the variable endotracheal tube head part 12 is in a round-hole-shape ventilation state, the diameter of the variable endotracheal tube head part 12 is the same as the diameter of the main tube of the telescopic stacked endotracheal tube 11.

Further, as shown in FIG. 1 and FIG. 2, in the efficient care-type variable endotracheal tube ventilation device provided in the embodiment of the present disclosure, the above variable endotracheal tube head part 12 is provided with a side opening 121, and the configuration of the side opening 121 can effectively improve the ventilation effect.

Further, as shown in FIG. 1 and FIG. 2, in the efficient care-type variable endotracheal tube ventilation device provided in the embodiment of the present disclosure, at least two side openings 121 may be included, and the configuration of a plurality of side openings 121 can enable ventilation flow to comply with the state of mixed flow in physiology better, so as to improve the aggregation capability of the ventilation flow, and ensure more concentrated ventilation flow.

Optionally, when there are three side openings 121, the three side openings 121 can be distributed in a triangle shape in a three-dimensional space, and this triangle may be an isosceles triangle, i.e. a triangle with two equal internal angles among three internal angles; or this triangle may be an equilateral triangle, i.e. a triangle with three internal angles of 60 °; or this triangle may be a right triangle, i.e. a triangle with one internal angle of 90 °among three internal angles; or this triangle may be an isosceles right triangle, i.e. a triangle with one internal angle of 90 ° and two other internal angles of 45 °among three internal angles; or this triangle may be a right triangle with an internal angle of 30 °, i.e. a triangle with one internal angle of 30 °, one internal angle of 60 °, and one internal angle of 90 °among three internal angles.

Of course, apart from the special triangles listed above, the triangle also may be a triangle with irregular degrees of internal angles, and this is not limited herein, but is within the scope of protection of the present disclosure.

Optionally, when there are four side openings 121, the four side openings 121 can be distributed in a cross shape in the three-dimensional space, and width and height of the cross shape are of the same length; of course, the width and height of the cross shape also may be provided with unequal lengths.

Optionally, when there are four side openings 121, the four side openings 121 can be distributed in a quadrilateral shape in the three-dimensional space, for example, the four side openings 121 are sequentially connected in a three-dimensional space to present a parallelogram distribution.

In practical application, in order to reduce the incision probability of the trachea, in the efficient care-type variable endotracheal tube ventilation device provided in the embodiment of the present disclosure, materials of the above main tube of the telescopic stacked endotracheal tube 11 are mainly metal and non-metal nano antibacterial materials, preferably an elastic film of PVC material with antibacterial property. The antibacterial property of the nano antibacterial materials and the structural advantages of the catheter can meet the requirement of tube retaining in ICU, and can prolong the tube-carrying duration for patients who need to be supported by a breathing machine, thus effectively reducing the incision probability of the trachea.

Of course, the material of the main tube of the telescopic stacked endotracheal tube 11 also may be other thin films meeting the requirements, for example, an elastic film of paper material with antibacterial property. Moreover, the above PVC material or paper material may have a thickness of 0.07 mm-0.17 mm.

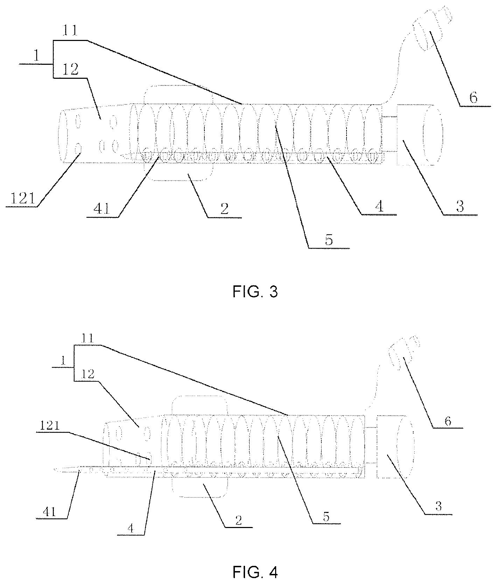

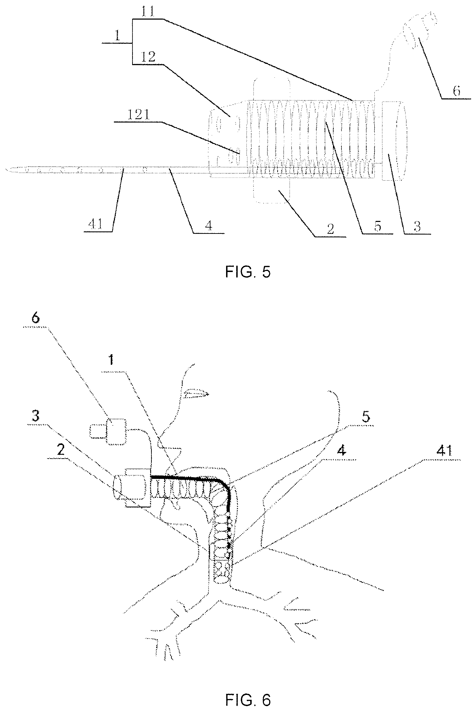

FIG. 3 is a structural schematic diagram of another efficient care-type variable endotracheal tube ventilation device provided in an embodiment of the present disclosure; FIG. 4 is a structural schematic diagram of another efficient care-type variable endotracheal tube ventilation device, with a first telescopic length, provided in an embodiment of the present disclosure; FIG. 5 is a structural schematic diagram of another efficient care-type variable endotracheal tube ventilation device, with a second telescopic length, provided in an embodiment of the present disclosure.

In the above, as shown in FIG. 3 - FIG. 5, in the efficient care-type variable endotracheal tube ventilation device provided in an embodiment of the present disclosure, an extended length of the above main tube of the telescopic stacked endotracheal tube 11 may be at least two times the length when the main tube of the telescopic stacked endotracheal tube is completely stacked; in specific use, the stacks at a distal end of the endotracheal tube are preferentially released when the endotracheal tube is moved in a direction from oral cavity towards the interior of the trachea, and a proximal end of the endotracheal tube is preferentially stacked when the endotracheal tube is retracted in a direction from the interior of the trachea towards the oral cavity, with a stacking thickness of less than 1.3 mm.

Specifically, as shown in FIG. 3 - FIG. 5, in the efficient care-type variable endotracheal tube ventilation device provided in an embodiment of the present disclosure, the above main tube of the telescopic stacked endotracheal tube 11 can be provided therein with a guide tube 4, and this guide tube 4 can be provided with rail holes 41 along an axial direction; moreover, the main tube of the telescopic stacked endotracheal tube 11 can be provided therein with an elastic support 5 along the axial direction, the elastic support 5 supports the main tube of the telescopic stacked endotracheal tube 11 in a circumferential direction, and this elastic support 5 preferably has a diameter of 0.1 mm-0.2 mm; in practical assembling and use, the guide tube 4 is provided in a manner of passing through the elastic support 5, and the endotracheal tube main body 1 can slide on the guide tube 4 in a telescopic stacking manner.

Further, in the efficient care-type variable endotracheal tube ventilation device provided in an embodiment of the present disclosure, the above guide tube 4 participates in ventilation, and a plurality of holes on a tube wall of the guide tube 4 can be all formed in a tear shape, participating in ventilation.

Furthermore, each of the above holes of the guide tube 4 formed in a tear shape may have a diameter of 1 mm in a head portion, and a diameter of 0.1-0.2 mm in a tail portion. Of course, specific values are not limited to these, while any other reasonable values are acceptable, which are not enumerated one by one herein.

Preferably, in practical production and manufacture, in the efficient care-type variable endotracheal tube ventilation device provided in an embodiment of the present disclosure, the above elastic support 5 may be a spring.

In practical application, as shown in FIG. 1 - FIG. 5, in the efficient care-type variable endotracheal tube ventilation device provided in an embodiment of the present disclosure, the above endotracheal tube plugging cuff 2 can be communicated with an inflation valve 6, so as to inflate the endotracheal tube plugging cuff 2 through the inflation valve 6.

The efficient care-type variable endotracheal tube ventilation device provided in the embodiment of the present disclosure can eliminate, to the greatest extent, the stimulation of the endotracheal tube to airway of the patient under the perioperative general anesthesia, effectively improve the tolerance of the patient, and is safer; besides, the patient with the tube can have a conversation while being awake, and in emergency circumstances, the endotracheal tube state can be restored or the endotracheal tube can be inserted again at any time, which is particularly suitable for patients who need to reserve the endotracheal tube when returning to the ward or ICU, and can prolong the tube-carrying duration for patients who need to be supported by a breathing machine, thus effectively reducing the incision probability of the trachea.

The present disclosure is designed on the basis of the theoretical research of the tool sequential extubation proposed by the inventor. The concept of tool sequential extubation (TSE) is: for the analepsia period of perioperative general anesthesia, a set of practical tools are researched, and with the tools, when the patient is changed from deep anesthesia to shallow anesthesia during the analepsia period of general anesthesia, the tools are switched such that the stimulation is changed from heavy to light so as to eliminate the choking cough reflex, reduce occurrence of complications, and improve safety of perioperative anesthesia. The efficient care-type variable endotracheal tube ventilation device provided in the present disclosure is suitable to all surgery patients, and is expected to become a common tool, especially suitable for avoiding tracheotomy of ICU patients and patients who need to carry the tube for a long period of time.

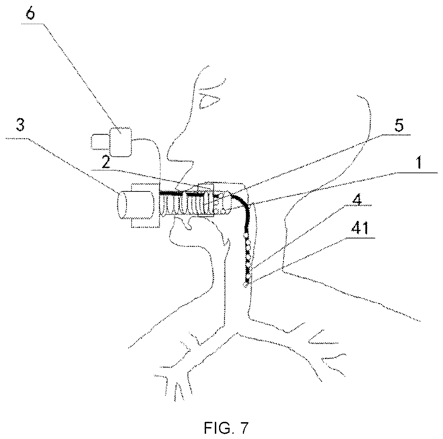

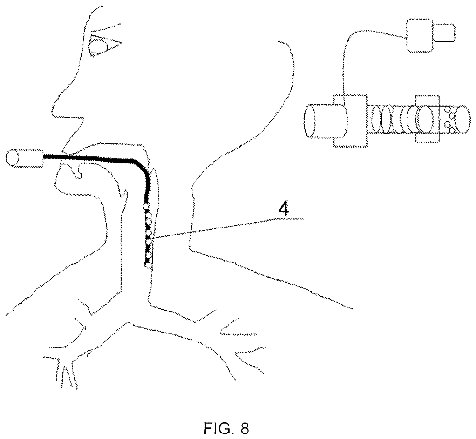

FIG. 6 is a structural schematic diagram of the efficient care-type variable endotracheal tube ventilation device, in an intubation state during anesthesia, provided in an embodiment of the present disclosure; FIG. 7 is a structural schematic diagram of the efficient care-type variable endotracheal tube ventilation device, in a state that the catheter is retracted back to the oral cavity during partial recovery of consciousness, provided in an embodiment of the present disclosure; and FIG. 8 is a structural schematic diagram of the efficient care-type variable endotracheal tube ventilation device, in a state that the catheter is removed during complete recovery of consciousness, provided in an embodiment of the present disclosure.

As shown in FIG. 6 - FIG. 8, in the using process of the present disclosure for clinical anesthesia, when a patient enters an anesthesia state from waking, spontaneous respiration of the patient disappears, and a front end of the variable endotracheal tube head part 12 is in a conical shape or stacked conical shape during intubation, which can reduce injury to oral tissues and glottis; after the efficient care-type variable endotracheal tube ventilation device passes through the glottis, the endotracheal tube plugging cuff 2 is inflated to prevent air leakage during respiratory support of the anesthesia machine, and the pressure on the wall of the endotracheal tube generated during inflation of the endotracheal tube plugging cuff 2 is supported by the elastic support 5 which is telescopically stacked, to prevent the endotracheal tube from collapsing to cause ventilation dysfunction; with the heating or inflating in the tube body, the conical shape or stacked conical shape of the variable endotracheal tube head part 12 becomes a cylindrical shape. After the operation is finished, the spontaneous respiration of the patient is resumed, anesthesia is in a relatively safe waking stage, the endotracheal tube plugging cuff 2 is deflated, and the endotracheal tube main body 1 is retracted back to the oral cavity, at this time, the respiratory rate RR of the patient is normal, and various ventilation indexes are in relatively safe ranges. The stimulation to the trachea is relieved after the endotracheal tube main body 1 is retracted back to the oral cavity, the incidence rate of choking cough of the patient is reduced, meanwhile, retracting the endotracheal tube main body 1 back into the oral cavity can keep compliant and smooth oral cavity ventilation, and prevent unsmooth airway caused by the collapse of oral tissues and glossocoma. Moreover, the guide tube 4 is reserved for air supply in the trachea, and the guide tube 4 is reserved for timely sending the endotracheal tube main body 1 back into the trachea to recover and control the ventilation state when the respiration of the patient in the analepsia period is inhibited after anesthesia, ensuring safety of the patient. When the patient is fully awake, the endotracheal tube main body 1 can be pulled out, reducing stimulation to the oral cavity, only the guide tube 4 needs to be reserved for supplying oxygen, and when the patient suffers from respiratory depression, the endotracheal tube main body 1 can be sent into the trachea through the guide tube 4, thus ensuring safety. For the patients who need to carry the tube in ICU, the main tube of the efficient care-type variable endotracheal tube ventilation device can be conveniently and safely sterilized and cleaned, prolonging the tube-carrying duration and reducing the probability of tracheotomy. The above use in the present disclosure is based on the tool sequential extubation proposed by the inventor.

## Claims

1. An efficient care-type variable endotracheal tube ventilation device, comprising: a telescopic endotracheal tube main body (1) with a variable length, wherein the endotracheal tube main body (1) comprises: a main tube of a telescopic stacked endotracheal tube (11),
wherein an endotracheal tube plugging cuff (2) is sheathed outside the endotracheal tube main body (1), and the endotracheal tube plugging cuff (2) is provided near a variable endotracheal tube head part (12), wherein the variable endotracheal tube head part (12) is connected to one end of the main tube of the telescopic stacked endotracheal tube (11); and
the other end of the main tube of the telescopic stacked endotracheal tube (11) is connected to an endotracheal tube connector (3),
**characterized in that**
the variable endotracheal tube head part (12) is formed of a temperature control material, and the variable endotracheal tube head part (12) is configured to develop to a cylindrical shape when a temperature reaches more than 30 degrees centigrade or 54.6 degrees Fahrenheit (12.6°C), and to be retracted back to a conical shape when the temperature reaches lower than 10 degrees centigrade or 18.6 degrees Fahrenheit (-7.4 °C); or the variable endotracheal tube head part (12) is in a stacked conical shape, and is configured in such a manner that the stacked conical shape of the variable endotracheal tube head part (12) is capable of developing to the cylindrical shape by an air flow aeration method.

2. The efficient care-type variable endotracheal tube ventilation device according to claim 1, wherein the variable endotracheal tube head part (12) is configured in such a way that, when in a cylindrical-shape ventilation state, a diameter of the variable endotracheal tube head part (12) is same as a diameter of the main tube of the telescopic stacked endotracheal tube (11).

3. The efficient care-type variable endotracheal tube ventilation device according to claim 1 or 2, wherein the variable endotracheal tube head part (12) is provided with side openings (121).

4. The efficient care-type variable endotracheal tube ventilation device according to claim 3, comprising at least two side openings (121).

5. The efficient care-type variable endotracheal tube ventilation device according to claim 4, wherein a plurality of the side openings (121) are distributed in a triangle shape in a three-dimensional space.

6. The efficient care-type variable endotracheal tube ventilation device according to claim 4, wherein a plurality of the side openings (121) are distributed in a cross shape in a three-dimensional space.

7. The efficient care-type variable endotracheal tube ventilation device according to claim 4, wherein a plurality of the side openings (121) are distributed in a quadrilateral shape in a three-dimensional space.

8. The efficient care-type variable endotracheal tube ventilation device according to any one of claims 1-7, wherein the telescopic stacked endotracheal tube has a main tube which is of an elastic film of PVC material or an elastic film of paper material, which has an antibacterial property; and
the PVC material or the paper material has a thickness of 0.07 mm-0.17 mm.

9. The efficient care-type variable endotracheal tube ventilation device according to claim 8, wherein an extended length of the main tube of the telescopic stacked endotracheal tube (11) is at least two times a length when the main tube of the telescopic stacked endotracheal tube (11) is completely stacked,
wherein the main tube of the endotracheal tube is configured in such a manner that stacks at a distal end of the endotracheal tube are preferentially released when the endotracheal tube is moved in a direction from an oral cavity towards an interior of trachea, and a proximal end of the endotracheal tube is preferentially stacked when the endotracheal tube is retracted in a direction from the interior of trachea towards the oral cavity, with a stacking thickness of less than 1.3 mm.

10. The efficient care-type variable endotracheal tube ventilation device according to any one of claims 1-9, wherein the main tube of the telescopic stacked endotracheal tube (11) is provided therein with a guide tube (4), and the guide tube (4) is provided with rail holes (41) along an axial direction;
the main tube of the telescopic stacked endotracheal tube (11) is provided therein with an elastic support (5) along an axial direction, and the elastic support (5) is configured to support the main tube of the telescopic stacked endotracheal tube (11) in a circumferential direction, and the elastic support (5) has a diameter of 0.1 mm-0.2 mm; and
the guide tube (4) is provided in a manner of passing through the elastic support (5), and the main body of the endotracheal tube is configured to be capable of sliding on the guide tube (4) in a telescopic stacking manner.

11. The efficient care-type variable endotracheal tube ventilation device according to claim 10, wherein the guide tube (4) is configured to participate in ventilation, and a plurality of holes on a tube wall of the guide tube (4) are all formed in a tear shape, for participating in ventilation.

12. The efficient care-type variable endotracheal tube ventilation device according to claim 11, wherein each of the holes formed in the tear shape of the guide tube (4) has a diameter of 1 mm in a head portion, and a diameter of 0.1-0.2 mm in a tail portion.

13. The efficient care-type variable endotracheal tube ventilation device according to any one of claims 10-12, wherein the elastic support (5) is a spring.

14. The efficient care-type variable endotracheal tube ventilation device according to any one of claims 1-13, wherein the endotracheal tube plugging cuff (2) is communicated with an inflation valve (6).

## Patentansprüche

1. Effiziente variable Endotrachealtubus-Belüftungsvorrichtung zur Versorgung, umfassend: einen teleskopisch ausziehbaren Endotrachealtubus-Hauptkörper (1) mit einer variablen Länge, wobei der Endotrachealtubus-Hauptkörper (1) umfasst: einen Haupttubus eines teleskopisch gestapelten Endotrachealtubus (11),
wobei eine Endotrachealtubus-Dichtmanschette (2) auf der Außenseite des Endotrachealtubus-Hauptkörpers (1) umhüllend angeordnet ist und die Endotrachealtubus-Dichtmanschette (2) neben einem variablen Endotrachealtubus-Kopfteil (12) vorgesehen ist,
wobei das variable Endotrachealtubus-Kopfteil (12) mit einem Ende des Haupttubus des teleskopisch gestapelten Endotrachealtubus (11) verbunden ist; und
das andere Ende des Haupttubus des teleskopisch gestapelten Endotrachealtubus (11) mit einem Endotrachealtubus-Konnektor (3) verbunden ist,
**dadurch gekennzeichnet, dass**
das variable Endotrachealtubus-Kopfteil (12) aus einem Temperaturkontrollmaterial besteht und das variable Endotrachealtubus-Kopfteil (12) ausgelegt ist, sich in eine zylindrische Gestalt zu entwickeln, wenn eine Temperatur mehr als 30 Grad Celsius oder 54,6 Grad Fahrenheit (12,6°C) erreicht, und wieder in eine konische Gestalt zurückgeführt zu werden, wenn die Temperatur weniger als 10 Grad Celsius oder 18,6 Grad Fahrenheit (-7,4°C) erreicht;
oder das variable Endotrachealtubus-Kopfteil (12) eine gestapelte konische Gestalt aufweist und derart ausgelegt ist, dass die gestapelte konische Gestalt des variablen Endotrachealtubus-Kopfteils (12) mittels eines Luftströmungsbelüftungsverfahrens sich in die zylindrische Gestalt entwickeln kann.

2. Effiziente variable Endotrachealtubus-Belüftungsvorrichtung zur Versorgung nach Anspruch 1, wobei das variable Endotrachealtubus-Kopfteil (12) derart ausgelegt ist, dass in einem zylindrischen Belüftungszustand ein Durchmesser des variablen Endotrachealtubus-Kopfteils (12) einem Durchmesser des Haupttubus des teleskopisch gestapelten Endotrachealtubus (11) gleicht.

3. Effiziente variable Endotrachealtubus-Belüftungsvorrichtung zur Versorgung nach Anspruch 1 oder 2, wobei das variable Endotrachealtubus-Kopfteil (12) Seitenöffnungen (121) aufweist.

4. Effiziente variable Endotrachealtubus-Belüftungsvorrichtung zur Versorgung nach Anspruch 3, umfassend mindestens zwei Seitenöffnungen (121).

5. Effiziente variable Endotrachealtubus-Belüftungsvorrichtung zur Versorgung nach Anspruch 4, wobei eine Vielzahl der Seitenöffnungen (121) in einer Dreiecksform in einem dreidimensionalen Raum verteilt ist.

6. Effiziente variable Endotrachealtubus-Belüftungsvorrichtung zur Versorgung nach Anspruch 4, wobei eine Vielzahl der Seitenöffnungen (121) in einer Kreuzform in einem dreidimensionalen Raum verteilt ist.

7. Effiziente variable Endotrachealtubus-Belüftungsvorrichtung zur Versorgung nach Anspruch 4, wobei eine Vielzahl der Seitenöffnungen (121) in einer viereckigen Form in einem dreidimensionalen Raum verteilt ist.

8. Effiziente variable Endotrachealtubus-Belüftungsvorrichtung zur Versorgung nach einem der Ansprüche 1-7, wobei der teleskopisch gestapelte Endotrachealtubus einen Haupttubus aufweist, der aus einer elastischen Folie aus PVC-Material oder einer elastischen Folie aus Papiermaterial besteht, die eine antibakterielle Eigenschaft aufweist; und
das PVC-Material oder das Papier-Material eine Dicke von 0,07 mm-0,17 mm aufweist.

9. Effiziente variable Endotrachealtubus-Belüftungsvorrichtung zur Versorgung nach Anspruch 8, wobei eine ausgezogene Länge des Haupttubus des teleskopisch gestapelten Endotrachealtubus (11) mindestens doppelt so lang ist wie eine Länge, wenn der Haupttubus des teleskopisch gestapelten Endotrachealtubus (11) vollständig gestapelt ist,
wobei der Haupttubus des Endotrachealtubus derart ausgelegt ist, dass Stapel an einem distalen Ende des Endotrachealtubus bevorzugt freigesetzt werden, wenn der Endotrachealtubus in einer Richtung von einer Mundhöhle zu einem Inneren der Luftröhre bewegt wird, und ein proximales Ende des Endotrachealtubus bevorzugt gestapelt wird, wenn der Endotrachealtubus in einer Richtung vom Inneren der Luftröhre zur Mundhöhle zurückgezogen wird, wobei eine Stapeldicke weniger als 1,3 mm beträgt.

10. Effiziente variable Endotrachealtubus-Belüftungsvorrichtung zur Versorgung nach einem der Ansprüche 1-9, wobei der Haupttubus des teleskopisch gestapelten Endotrachealtubus (11) ein Führungsrohr (4) darin aufweist, und das Führungsrohr (4) Schienenbohrungen (41) entlang einer axialen Richtung aufweist;
der Haupttubus des teleskopisch gestapelten Endotrachealtubus (11) eine elastische Stütze (5) darin entlang einer axialen Richtung aufweist, und die elastische Stütze (5) zur Unterstützung des Haupttubus des teleskopisch gestapelten Endotrachealtubus (11) in einer Umfangsrichtung ausgelegt ist, und die elastische Stütze (5) einen Durchmesser von 0,1 mm-0,2 mm aufweist; und
das Führungsrohr (4) auf eine solche Weise bereitgestellt ist, dass es durch die elastische Stütze (5) verläuft, und der Hauptkörper des Endotrachealtubus ausgelegt ist, auf dem Führungsrohr (4) auf eine teleskopisch stapelnde Weise gleiten zu können.

11. Effiziente variable Endotrachealtubus-Belüftungsvorrichtung zur Versorgung nach Anspruch 10, wobei das Führungsrohr (4) zur Beteiligung an der Belüftung ausgelegt ist, und eine Vielzahl von Löchern an einer Rohrwand des Führungsrohrs (4) zur Beteiligung an der Belüftung alle tränenförmig ausgebildet sind.

12. Effiziente variable Endotrachealtubus-Belüftungsvorrichtung zur Versorgung nach Anspruch 11, wobei jedes der tränenförmig ausgebildeten Löcher des Führungsrohrs (4) einen Durchmesser von 1 mm in einem Kopfabschnitt und einen Durchmesser von 0,1-0,2 mm in einem Endabschnitt aufweist.

13. Effiziente variable Endotrachealtubus-Belüftungsvorrichtung zur Versorgung nach einem der Ansprüche 10-12, wobei die elastische Stütze (5) eine Feder ist.

14. Effiziente variable Endotrachealtubus-Belüftungsvorrichtung zur Versorgung nach einem der Ansprüche 1-13, wobei die Endotrachealtubus-Dichtmanschette (2) mit einem Aufblasventil (6) in Verbindung steht.

## Revendications

1. Dispositif de ventilation de tube endotrachéal variable de type soin efficace, comprenant :
un corps principal de tube endotrachéal télescopique (1) avec une longueur variable, le corps principal de tube endotrachéal (1) comprenant : un tube principal d'un tube endotrachéal empilé télescopique (11),
un manchon d'obturation de tube endotrachéal (2) étant gainé à l'extérieur du corps principal de tube endotrachéal (1), et le manchon d'obturation de tube endotrachéal (2) étant prévu près d'une partie de tête de tube endotrachéal variable (12), la partie de tête de tube endotrachéal variable (12) étant raccordée à une extrémité du tube principal du tube endotrachéal empilé télescopique (11) ; et
l'autre extrémité du tube principal du tube endotrachéal empilé télescopique (11) étant raccordée à un raccord de tube endotrachéal (3),
**caractérisé en ce que**
la partie de tête de tube endotrachéal variable (12) est constituée d'un matériau de régulation de température, et la partie de tête de tube endotrachéal variable (12) est configurée pour se développer en une forme cylindrique lorsqu'une température atteint plus de 30 degrés centigrades ou 54,6 degrés Fahrenheit (12,6 °C), et pour être rétractée en une forme conique lorsque la température atteint moins de 10 degrés centigrades ou 18,6 degrés Fahrenheit (-7,4 °C) ;
ou la partie de tête de tube endotrachéal variable (12) a une forme conique empilée, et est configurée de telle manière que la forme conique empilée de la partie de tête de tube endotrachéal variable (12) peut se développer en une forme cylindrique par un procédé d'aération par flux d'air.

2. Dispositif de ventilation de tube endotrachéal variable de type soin efficace selon la revendication 1, la partie de tête de tube endotrachéal variable (12) étant configurée de telle sorte que, lorsqu'elle est dans un état de ventilation de forme cylindrique, un diamètre de la partie de tête de tube endotrachéal variable (12) est le même qu'un diamètre du tube principal du tube endotrachéal empilé télescopique (11).

3. Dispositif de ventilation de tube endotrachéal variable de type soin efficace selon la revendication 1 ou 2, la partie de tête de tube endotrachéal variable (12) étant pourvue d'ouvertures latérales (121).

4. Dispositif de ventilation de tube endotrachéal variable de type soin efficace selon la revendication 3, comprenant au moins deux ouvertures latérales (121).

5. Dispositif de ventilation de tube endotrachéal variable de type soin efficace selon la revendication 4, une pluralité d'ouvertures latérales (121) étant réparties en forme de triangle dans un espace tridimensionnel.

6. Dispositif de ventilation de tube endotrachéal variable de type soin efficace selon la revendication 4, une pluralité d'ouvertures latérales (121) étant réparties en forme de croix dans un espace tridimensionnel.

7. Dispositif de ventilation de tube endotrachéal variable de type soin efficace selon la revendication 4, une pluralité d'ouvertures latérales (121) étant réparties en forme de quadrilatère dans un espace tridimensionnel.

8. Dispositif de ventilation de tube endotrachéal variable de type soin efficace selon l'une quelconque des revendications 1 à 7, le tube endotrachéal empilé télescopique ayant un tube principal qui est constitué d'un film élastique en matériau PVC ou d'un film élastique en matériau papier, qui a une propriété antibactérienne ; et
le matériau PVC ou le matériau papier ayant une épaisseur de 0,07 mm à 0,17 mm.

9. Dispositif de ventilation de tube endotrachéal variable de type soin efficace selon la revendication 8, une longueur étendue du tube principal du tube endotrachéal empilé télescopique (11) étant au moins deux fois une longueur lorsque le tube principal du tube endotrachéal empilé télescopique (11) est complètement empilé,
le tube principal du tube endotrachéal étant configuré de telle manière que les empilements au niveau d'une extrémité distale du tube endotrachéal sont préférentiellement libérés lorsque le tube endotrachéal est déplacé dans une direction allant d'une cavité orale vers l'intérieur de la trachée, et une extrémité proximale du tube endotrachéal étant préférentiellement empilée lorsque le tube endotrachéal est rétracté dans une direction allant de l'intérieur de la trachée vers la cavité orale, avec une épaisseur d'empilement inférieure à 1,3 mm.

10. Dispositif de ventilation de tube endotrachéal variable de type soin efficace selon l'une quelconque des revendications 1 à 9, le tube principal du tube endotrachéal empilé télescopique (11) étant prévu à l'intérieur d'un tube de guidage (4), et le tube de guidage (4) étant pourvu de trous de rail (41) le long d'une direction axiale ;
le tube principal du tube endotrachéal empilé télescopique (11) étant pourvu d'un support élastique (5) le long d'une direction axiale, et le support élastique (5) étant configuré pour supporter le tube principal du tube endotrachéal empilé télescopique (11) dans une direction circonférentielle, et le support élastique (5) ayant un diamètre de 0,1 mm à 0,2 mm ; et le tube de guidage (4) étant fourni de manière à passer à travers le support élastique (5), et le corps principal du tube endotrachéal étant configuré pour pouvoir glisser sur le tube de guidage (4) d'une manière empilée télescopique.

11. Dispositif de ventilation de tube endotrachéal variable de type soin efficace selon la revendication 10, le tube de guidage (4) étant configuré pour participer à la ventilation, et une pluralité de trous sur une paroi de tube du tube de guidage (4) étant tous formés en forme de larme, pour participer à la ventilation.

12. Dispositif de ventilation de tube endotrachéal variable de type soin efficace selon la revendication 11, chacun des trous formés dans la forme de larme du tube de guidage (4) ayant un diamètre de 1 mm dans une partie de tête, et un diamètre de 0,1 à 0,2 mm dans une partie de queue.

13. Dispositif de ventilation de tube endotrachéal variable de type soin efficace selon l'une quelconque des revendications 10 à 12, le support élastique (5) étant un ressort.

14. Dispositif de ventilation de tube endotrachéal variable de type soin efficace selon l'une quelconque des revendications 1 à 13, le manchon d'obturation de tube endotrachéal (2) étant mis en communication avec une valve de gonflage (6).
